# EUROPEAN PATENT SPECIFICATION

(11) **EP 0 945 462 B1**
(45) Date of publication and mention of the grant of the patent: **02.08.2006**
(21) Application number: 98302287.2
(22) Date of filing: 25.03.1998
(51) Int. Cl.: C07K 14/35, C12Q 1/68

(54) **Mycobacterium tuberculosis specific DNA fragment**
Mycobacterium tuberculosis spezifisches DNS-Fragment
Fragment d'ADN spécifique du mycobacterium tuberculosis

(43) Date of publication of application: 29.09.1999
(73) Proprietor: COUNCIL OF SCIENTIFIC AND INDUSTRIAL RESEARCH, New Delhi 110 001 (IN)
(72) Inventor: Srivastava, Ranjana, Lucknow-1 (IN); Kumar, Deepak, Lucknow-1 (IN); Srivastava, Brahm Shanker, Lucknow-1 (IN)
(74) Representative: Grant, Anne Rosemary

(56) References cited:
- WO-A-91/19008
- WO-A-95/21941
- WO-A-97/35985
- PHILIPP W J ET AL: "AN INTEGRATED MAP OF THE GENOME OF THE TUBERCLE BACILLUS, MYCOBACTERIUM TUBERCULOSIS H37RV, AND COMPARISON WITH MYCOBACTERIUMLEPRAE" PROCEEDINGS OF THE NATIONAL ACADEMY OF SCIENCES OF USA, vol. 93, April 1996, pages 3132-3137, XP002042810
- THIERRY D ET AL: "IS6110, AN IS-LIKE ELEMENT OF MYCOBACTERIUM TUBERCULOSIS COMPLEX" NUCLEIC ACIDS RESEARCH, vol. 18, no. 1, 11 January 1990, page 188 XP000371715
- KOLK A H J ET AL: "DETECTION OF MYCOBACTERIUM TUBERCULOSIS IN CLINICAL SAMPLES BY USING POLYMERASE CHAIN REACTION AND A NONRADIOACTIVE DETECTION SYSTEM" JOURNAL OF CLINICAL MICROBIOLOGY, vol. 30, no. 10, October 1992, pages 2567-2575, XP000619678
- SAHADEVAN ET AL.: "Restriction fragment length polymorphism typing of clinical isolates of Mycobacterium tuberculosis from patients with pulmonary tuberculosis in Madras, India, by use of a direct-repeat probe" JOURNAL OF CLINICAL MICROBIOLOGY, vol. 33, no. 11, November 1995, pages 3037-3039, XP002076898

## Description

This invention relates to a *Mycobacterium tuberculosis* specific DNA fragment containing IS like and repetitive sequences, an *ex vivo* method of production of such a DNA fragment and the *ex vivo* use of such a DNA fragment, for example, to rapidly diagnose *Mycobacterium tuberculosis* infection in clinical samples, and to identify clinical isolates of *Mycobacterium tuberculosis.* The DNA fragment may be used to determine information about the epidemiology of *Mycobacterium tuberculosis* infection. Specifically this invention relates to the use of sequence specific DNA fragments to diagnose Mycobacterium tuberculosis and strains of *Mycobacterium tuberculosis*. A purpose of the study of the epidemiology of tuberculosis is to distinguish the genetic diversity of the causative agent *Mycobacterium tuberculosis* and to obtain information about strain to strain variability. This can be achieved by molecular epidemiological methods including DNA fingerprinting and restriction fragment length polymorphism (RFLP) analysis. Such approaches, can aid the investigation of point source outbreaks, transmission, pathogenesis and may be employed as a marker of strain typing.

Tuberculosis (TB) is a major cause of infectious mortality. According to a recent WHO report, the number of deaths attributed to TB was a larger number in 1995 than in any other year in history (Moran, N. 1996. WHO Issues Another Gloomy Report. Nature Medicine 4:377). Tuberculosis remains widespread worldwide and constitutes a major health problem particularly in developing countries. One third of the total world's population (nearly two billion people) is infected with *Mycobacterium tuberculosis* out of which 5 to 10% develop the disease. TB causes more than 3 million deaths per year and recently WHO has predicted that 30 million people will die of TB in the next ten years (Joint International Union Against Tuberculosis and World Health Organization Study Group. Tubercl 63:157-169, 1982).

Tuberculosis is caused by a gram positive acid fast bacterium *Mycobacterium tuberculosis* or *M. bovis,* which are the tubercle bacilli of the family of *Mycobacteriaceae. M. bovis* is a species which causes tuberculosis in cattle and can be transmitted to humans and other animals in which it causes tuberculosis. At present nearly all tuberculosis in humans is caused by *Mycobacterium tuberculosis*. Infections occasionally result from other species of mycobacteria that are common environmental saprophytes. These species have been collectively termed as MOTT (Mycobacteria other than typical tubercle), environmental or tuberculoid bacilli. The difference between the two infections is that infection with *Mycobacterium tuberculosis* is always transmitted from host to host. In contrast, human beings infected with other mycobacteria rarely transmit the disease.

Hence the essential component of any tuberculosis control program is containment of the disease. Identification of infected individuals, especially those most likely to transmit viable bacilli, comes as a first priority in strategies for tuberculosis control. Early and timely diagnosis of tuberculosis is essential for identifying individuals carrying the bacilli. Therefore a need has arisen for a method of diagnosis of tuberculosis which is rapid, sensitive and specific. Routine diagnostic methods used for identification of *Mycobacterium tuberculosis* includes acid fast smear tests in clinical samples like sputum, tests based on growth of bacilli of specific media and differential biochemical tests. The culture of mycobacteria from clinical samples is the most reliable and provides for definite diagnosis of tuberculosis. Although 100% specific, it takes six to eight weeks due to slow growth of organisms and further biochemical testing before identification can be made (Heifests, LB. and Good, R.C. 1994. Current Laboratory Methods for the Diagnosis of Tuberculosis. Tuberculosis Pathogenesis, Protection and Control (ed. B.R. Bloom) ASM Washington DC, pp. 85-110).

Methods based on antigen and antibody detection in body fluids and more recently nucleic acid probes (sequence specific DNA fragments) have been developed as reagents for rapid diagnosis and monitoring of the epidemiology of tuberculosis (Young, D.B. and Mehlert, A. 1989, Serology of Mycobacteria: Characterization of Antigens Recognized by Monoclonal Antibodies. Rev. Infec. Dis. 12: S431-S435; Pfyfer, G.E., Kisling, P., Jahn, E.M.I., Martin, H., Salfinger; W.M. and Weber, R. 1996. Diagnostic Performance of Amplified *Mycobacterium* Tuberculosis Direct Test with Cerebrospinal Fluid, Other Non Respiratory and Respiratory Specimens, J. Clin. Microbiol. 34:834-841).

The DNA probes utilize a wide array of sequences from *Mycobacterium* tuberculosis ranging from whole genomic DNA, to a single copy sequence, and to repetitive DNA elements. When evaluated directly on clinical samples they have proved to be highly specific, sensitive and dramatically reduce the time for diagnosis of tuberculosis (Kiehn, T.E. 1993. The Diagnostic Mycobacteriology Laboratory of the 1990's. Clin. Infect. Dis. 17 (suppl.2) S447-S454).

Several sequence specific probes have been used as targets for identification of Mycobacterium *tuberculosis* by amplification of specific sequences by PCR.

IS6110 is an IS element present in members of *Mycobacterium* tuberculosis complex *(Mycobacterium tuberculosis, M. bovis, M. africanum*, and *M.* microti).

Different regions of IS6110 have been amplified using different sets of primers for PCR based diagnosis like the 123 base pair (bp) or 245 bp region (Einsenach, K.D., Cave, M.D., Bates, J.H. and Crawford, J.T. 1990. Polymerase chain reaction amplification of repetitive DNA sequence specific for *Mycobacterium tuberculosis*. J. Infect. Dis. 161:977-981; Kolk, A.H.J., Schuitema, A.R.J., Kuijper, S., Vanheeuwen J., Hermans, P.W.M., Van Embden, J.D.A. Hartskeerl, R.A. 1992, Detection of *Mycobacterium tuberculosis* in clinical samples by using polymerase chain reaction and a non radioactive detection system. J. Clin. Microbiol. 30:2567-2575).

IS6110 has some disadvantages. Several Mycobacterium *tuberculosis* strains with one copy or no copy of IS6110 have been reported (Sahadevan, R., Narayanan, S. Paramsivam, C.N-, Prabhakar, R. and Narayanan, P.R. 1995. Restriction fragment length polymorphism typing of clinical isolates of *Mycobacterium tuberculosis* from patients with pulmonary tuberculosis in Madras, India by use of direct repeat probe. J. Clin. Microbiol. 33:3037-3039: Van Soolingen, D., Dehass, P.E.W., Hermans, P.W.M. Groenen, P.M.A. and Van Embden, J.D.A. 1993. Comparison of various repetitive DNA elements as genetic markers for strain differentiation and epidemiology of *Mycobacterium tuberculosis*. J. Clin. Microbiol. 31: 1987-1995). Thus the repertoire of *Mycobacterium tuberculosis* strains present all over the world may not be selected/amplified using a single repetitive element or one DNA probe specific to *Mycobacterium tuberculosis.* The search for newer DNA probes is a constant requirement.

It is an object of this invention to detect *Mycobacterium tuberculosis* in a clinical sample.

It is another object of this invention to describe a DNA fragment containing an IS like sequence and repetitive sequences.

It is a further object of this invention to use a DNA fragment containing an IS like sequence and repetitive sequences to detect *Mycobacterium* tuberculosis *ex vivo.*

It is yet another object of this invention to describe an *ex vivo* method for production of a DNA fragment containing an IS like sequence and repetitive sequences.

It is another object of this invention to use a 1291 base pair sequence having an IS like sequence and repetitive sequences or a portion or fragment of the 1291 base pair sequence to detect *Mycobacterium tuberculosis ex vivo.*

It is a further object of this invention to construct a sequence specific DNA probe for the identification of *Mycobacterium tuberculosis* present in clinical samples.

It is another object of this invention to develop a sequence specific DNA probe for identification of clinical isolates of *Mycobacterium tuberculosis* in order to distinguish it from other mycobacteria which may be present in the clinical samples.

The sequence specific DNA probes of the invention may be used for monitoring the epidemiology of tuberculosis, for the detection of polymorphism in clinical isolates of *Mycobacterium tuberculosis* or to provide an IS element for transposition, mutagenesis and gene inactivation in mycobacteria for the investigation of pathogenesis, virulence and vaccine development.

Aspects of the invention are as set forth in the claims.

This invention relates to *Mycobacterium tubercu*losis specific DNA fragment containing an IS like sequence and repetitive sequences, an *ex vivo* method of production of such a DNA fragment and the *ex vivo* use of such a DNA fragment, for example, to rapidly diagnose *Mycobacterium tuberculosis* infection in clinical samples, to identify clinical isolates of *Mycobacterium tuberculosis* and to track the epidemiology of *Mycobacterium tuberculosis* infection. Specifically this invention relates to the *ex vivo* use of a sequence specific DNA fragment to diagnose tuberculosis and strains of *Mycobacterium tuberculosis*. The *Mycobacterium tuberculosis* specific DNA fragment consists of 1291 base pair (bp) sequence which contains an IS like element and several repeat DNA sequences.

When this DNA fragment is incubated with clinical samples which may contain mycobacteria of interest, the presence of *Mycobacterium tuberculosis* can be detected by specificity of hybridization. The DNA primers designed from the nucleotide sequence of this 1291 bp fragment may be incubated with clinical samples to amplify the DNA of *Mycobacterium tuberculosis* present in the clinical samples. Hence if the samples contain *Mycobacterium tuberculosis* it may be diagnosed specifically. The DNA fragment when hybridized with genomic DNA of'clinical isolates of *Mycobacterium tuberculosis* by Southern hybridization, specifically hybridizes with *Mycobacterium tuberculosis* isolates and displays polymorphism. Hence, the fragment can be used in the specific diagnosis of *Mycobacterium tuberculosis* infections and to determine information about the epidemiology of *Mycobacterium tuberculosis* infections.

Mycobacteria can be detected in samples of sputum, cerebrospinal fluid, pleural fluid, urine, ascitic fluid, gastric samples, bronchial lavage, pericardial fluid, pus or lymph node aspirate.

One way to produce the *Mycobacterium tuberculosis* specific DNA fragment of the invention, is to use a genomic library of *Mycobacterium tuberculosis* DNA which may be constructed in a plasmid or phage lambda gtll vector. The library of the genomic DNA fragments may be screened with genomic DNA of *Mycobacterium tuberculosis* as a probe to select the fragments which rapidly hybridize to *Mycobacterium tuberculosis* genomic DNA.

The detection of positive hybridization signals within 60 minutes may indicate that the clones may contain repeat sequences in the DNA fragment of *Mycobacterium tuberculosis*.

The clones thus obtained may further be screened by DNA-DNA hybridization with genomic DNA of mycobacteria other than *Mycobacterium tuberculosis* used as probes. The clones not producing any hybridization signals may be selected as containing *Mycobacterium tuberculosis* specific DNA fragments.

The DNA fragment of this invention may be used by incubating it with samples which may contain the specific mycobacteria of interest (*Mycobacterium* tuberculosis). If *Mycobacterium tuberculosis* is present, then the DNA fragment will hybridize with the DNA of *Mycobacterium tuberculosis* which can be detected for example by dot blot or Southern hybridization assay.

The nucleotide sequence of the specific DNA fragment may be determined and DNA primers may be designed for specific amplification of *Mycobacterium tuberculosis* DNA. The specific DNA primers may be added into the clinical samples to amplify *Mycobacterium tuberculosis* DNA if present in the sample. The amplified product may be visualized by for example agarose gel electrophoresis and hybridization with the specific DNA fragment of the invention. The polymerase chain reaction (PCR) amplification as well as hybridization with the specific DNA fragment of the invention may indicate that the sample contains *Mycobacterium tuberculosis* and hence may be used in detection and diagnosis of tuberculosis. By hybridization with the specific DNA fragment of the invention, epidemiology of tuberculosis and *Mycobacterium tuberculosis* may be traced as the fragment contains several repeat sequences and thus strain polymorphism of *Mycobacterium tuberculosis* may be determined.

### BRIEF DESCRIPTION OF THE DRAWINGS

**Figure 1.** Screening of recombinant lambda gtll library of *Mycobacterium tuberculosis* with DIG-labeled denatured genomic DNA of *Mycobacterium tuberculosis* by DNA hybridization. Positive plaques gave hybridization signals within 60 min and appeared blue in color.

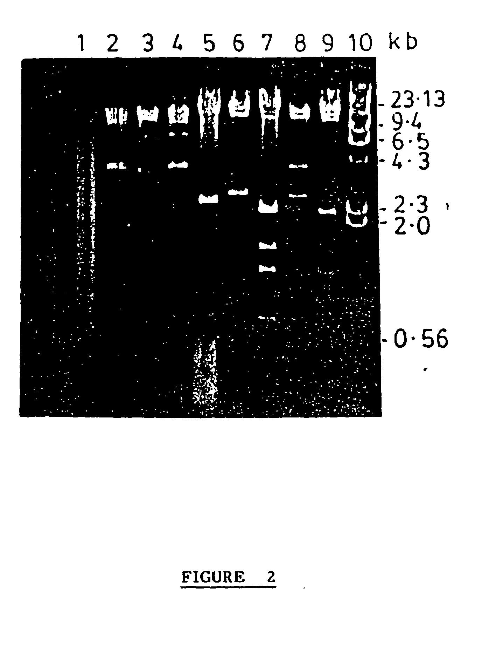

**Figure 2.** Agarose (1%) gel electrophoresis analysis of EcoRI digests of recombinant lambda clones. The DNA from recombinant phages was isolated, digested with EcoRI restriction enzyme and electrophoresed. Lambda HinDIII (BRL) was used a molecular weight marker. Lane 2: Clone C8; Lane 3: Clone C10; Lane 4: Clone C16; Lane 5: Clone C33; Lane 6: Clone C38; Lane 7: Clone C41; Lane 8: Clone C60; Lane 9: Clone C70; Lane 10: Lambda HindIII.
**Figure 3.** Southern hybridization of EcoRI digests of recombinant lambda clones with DIG-labeled denatured genomic DNA of *Mycobacterium tuberculosis*. The recombinant DNA clones shown on the gel are: Lane 1: Clone C70; Lane 2: Clone C60; Lane 3: Clone C41; Lane 4:Clone C38; Lane 5: Clone C33; Lane 6: Clone C16; Lane 7: Clone C10; Lane 8: Clone C8.
**Figure 4.** Restriction map of the 4.2 kb DNA fragment of *Mycobacterium tuberculosis* from Clone C8. The DNA was mapped with restriction endonucleases.
**Figure 5.** Restriction map of the 1291 bp StuI-StuI fragment of *Mycobacterium tuberculosis* from clone C8.

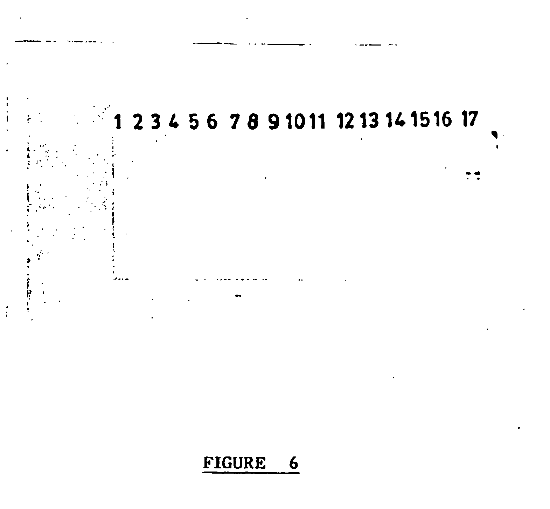

**Figure 6.** DNA hybridization of the 1291 bp DNA fragment from recombinant clone CD8 with other mycobacterial DNA. Southern hybridization of EcoRI digests of chromosomal DNA of various mycobacterial species (Lanes 1-7) with the DIG labeled denatured 1291 bp DNA fragment. Lane 1: *M. smegmatis;* Lane 2: *M. phlei;* Lane 3: *M.* avium *intracellulare* complex; Lane 4: *M. scrofulaceum;* Lane 5: *M. fortuitum;* Lane 6: *M. kansasii;* Lane 7: *M. gordonae*; Lane 8: *M.* asiaticum; Lane 9: *M. aurum;* Lane 10: *M.* chitae; Lane 11: *M. chelonae;* Lane 12: *M. xenopi;* Lane 13: *M. triviale;* Lane 14: *M. marinum*; Lane 15: *M. microti*; Lane 16: *M. flavescens;* Lane 17: *Mycobacterium tuberculosis*.
**Figure 7**. Dot-blot hybridization of non-mycobacterial DNA with the DIG-labeled denatured 1291 bp DNA fragment; 1. Salmonella typhimurium; 2. Enterobacter alginii; 3. Klebsiella pneumoniae; 4. Pseudomonas aeruginosa; 5. Proteus vulgaris; 6- Staphylococcus aureus; 7. Escherichia coli; 8. Edwardsiella sp.; 9. *Mycobacterium tuberculosis*; 10. Human placental DNA.
**Figure 8.!** DNA hybridization of the 1291 bp Stul-StuI fragment with StuI digested genomic DNA of clinical isolates of *Mycobacterium tuberculosis*.
**Figure 9.** Nucleotide sequence of the 1291 bp StuI-StuI fragment of *Mycobacterium tuberculosis*.
**Figure 10.** Deduced amino acid sequence from the nucleotide sequence (as in Fig. 9) of the 1291 bp StuI-StuI DNA fragment of *Mycobacterium tuberculosis.*

The invention is directed to a *Mycobacterium tuberculosis* specific DNA fragment, *ex vivo* methods of producing such a DNA fragment, characterization of such a DNA fragment, nucleotide sequence of such a DNA fragment and the ex *vivo* use of the DNA fragment or portion thereof for the rapid diagnosis of *Mycobacterium tuberculosis* infection in clinical samples to identify clinical isolates of *Mycobacterium tuberculosis* and to monitor the epidemiology of *Mycobacterium tuberculosis* infection as set forth in the claims. Specifically, this invention relates to the *ex vivo* of a sequence specific DNA fragment to diagnose tuberculosis and strains of *Mycobacterium tuberculosis.*

The invention relates to the *ex vivo* production and identification of a DNA fragment which contains an IS like sequence and various repeat sequences unique to *Mycobacterium tuberculosis*, which is the major etiologic agent of tuberculosis. In particular, it is based on the isolation of a *Mycobacterium tuberculosis* specific DNA fragment containing repetitive sequences using genomic DNA of *Mycobacterium tuberculosis* as a probe. Repetitive sequences are those which are present in several copies in the genome and give positive hybridization with a genomic DNA probe within a short detection period.

As used herein the IS like element is intended to refer to an insertion sequence. An insertion sequence is a small, transposable element in bacteria (a small transposon) that can insert into several sites in a genome. Insertion sequences function in the transposition of segments which they flank. They usually contain genes coding for proteins that function in their transposition but contain no other genes and have no other known effect than to function in transposition. The termini of each insertion sequence consist of inverted repeats.

Accordingly, the isolated DNA fragment of *Mycobacterium tuberculosis* comprises 1291 base pairs, the DNA fragment having following nucleotide sequence (SEQ ID NO:1:):

AGGCCTCGGT GACCGTGATC ATGTTGCCGC CGAAGGTCAT TACGTTGTGT ACGTCAATGA
CCATCTGCTC GTTGTTTATG GGGATGAATC GGGAGTGGTG ACCGAGAGAT CGATGGCGAA
TCTGGCCCTG GTTATCGCCC GCCACCAAGA AGCCATTGTT GAAGTCGCCC GTGTCGAAAG
CGCCGGTATT GACGTTGCCG GGATTGAAGA AGCCGGTGTT GGTGTCACCC GGGTTATAGC
TGCCGGTATT GGTGTCACCC ACGTTGAAGT TGCCGGTGTT GGTGTTACCG ACGTTGAAGC
CGCCGGTGTT GTAGCTGCCC GTGTTGTAGA AGCCCGTGTT GAAGTCGCCG GCGTTGAGGA
TGCCCGTGTT GTAGCTGCCA GCATTGAGGA TGCCGGTATT GTCGGTACCC GGGTTCCCGA
TACCCCAGTT CCCGGTGCCC GAGTTTGCGA TGCCGACGTT TCCGGTGCCC GCGTTGAAGA
TGCCAACGTT ATTGGTGCCC GAATTGAACA GGCCGCTGTT GCCGGTGCCC GAGTTCCAGC
CGCTAGCAAT ATTGAAGCCC TGCTGGTTGT CGCCGGACAG CCCGATGCCG ATGTTGTTGT
TGCCGGTGTT GGCGAACCCG ATGTTGTTGT TGCCGGTGTT GGCAAAGCCT TGGTTGAAGT
CGCCCGCGTT CCCGAAGCCG ACGTTGTAGT CGCCGACGTT TCCAAAACCG ATGTTGTAGA
TCCCGAGGTT TCCGGATCCG ATGTTGTAGT TTCCCAGGCT CCCGGAACCG ACATTGAATA
CTCCGATGTT TCCACTGCCG ATATTGAAGC TGCCGACGTT GCCGCTGCCC AAGATGTTTT
GGCTGCCGAG GTTGCCGCTG CCAAGGATGT TGAAGTCACC GACGTTTCCG CTGCCGAGAA
TGTTGTAATT GCCGATGTTG GCGTTGCCGA GAATGTTCAC GACGCCCCGG TTTGCCAGGC
CGAGATTGAA GACCGGTGGG CCACCGAAAA ATCCCGACAT GTTGCTTCCG GTGTTGAAGA
AGCCCGAGAT CAAGGCCGGC GTTGTGATGG CCACCAGGCT CATGTTGAAC AAACCCGATA
CGGTGTTGCC CGAGTTGATC ACGCCCGATA CCAGCACGCC CGCGTTTGCC AGGCCGGAGT

TACCGATGGC CCCCGACGAA GAGTGGAAGA AGCCAGAATT GTTGGCACCG GAGTTCAGGA
AGCCGGACGC GCTACCGGCA CCGCTGTTGA AGAATCCCGA CGACGGCGCA CTGGTCGAGT
TGAAGAAGCC GGGCTCCCGA AAATCAGGCC T

The invention relates also to sequences complementary to this 1291 base sequence and to sequences which are substantially homologous with or which hybridise to any of the aforesaid sequences.

"Substantially homologous" as used herein includes those sequences having a sequence identity of approximately 95%, 97%, 98% or 99% or more. This includes functionally-equivalent allelic variants and related sequences modified by single or multiple base substitution, addition or deletion, whilst still being specific for *Mycobacterium tuberculosis.* Functionally equivalent covers nucleic acid sequences which are likewise determinant and specific to *Mycobacterium tuberculosis.* Hybridisation' as used herein defines those sequences binding under non-stringent conditions (6 x SSC/50% formamide at room temperature) and washed under conditions of low stringency (2 x SSC, room temperature, preferably 2 x SSC, 42°C) or conditions of higher stringency e.g. 2 x SSC, 65°C (where SSC = 0.15M NaCl, 0.015M sodium citrate, pH 7.2), as well as those which, but for the degeneracy of the code, would hybridise with the aforementioned sequence. Sequences of the invention and for use in methods of the invention however extend to sequences which bind under the non-stringent conditions and are washed under conditions of higher stringency as defined above.

In another embodiment of the invention, there is an *ex vivo* method for the production and identification of a DNA fragment of *Mycobacterium tuberculosis* comprising 1291 base pairs. One such method comprises the following steps:
(a) constructing a genomic library of *Mycobacterium tuberculosis* in a vector including phage lambda gtll or a plasmid;
(b) screening the genomic library of *Mycobacterium tuberculosis* with genomic DNA of *Mycobacterium tuberculosis* as probe to select DNA fragments capable of specifically and rapidly hybridizing to *Mycobacterium tuberculosis* genomic DNA;
(c) labeling the probe with a label such as digoxigenin, biotin, radiolabelled with ³²P or alkaline phosphatase;
(d) isolating clones including repetitive DNA fragments with positive hybridization signals appearing within 10 to 60 minutes; and
(e) further screening of the clones isolated above for validation by DNA-DNA hybridization with generic DNA of mycobacteria other than *Mycobacterium tuberculosis* as probe to confirm that the clone selected above does not produce any hybridization signals with DNA of mycobacteria other than *Mycobacterium tuberculosis.*

The DNA fragment identified in this process can be used for the detection of *Mycobacterium tuberculosis* by incubating the isolated DNA fragment with samples which may contain specific mycobacteria (*Mycobacterium tuberculosis)* and detecting the presence or absence of *Mycobacterium tuberculosis.*

If DNA of *Mycobacterium tuberculosis* is present then the DNA fragment will hybridize with the DNA of *Mycobacterium tuberculosis*.

To produce a sequence specific DNA fragment of *Mycobacterium tuberculosis*, a genomic library of *Mycobacterium tuberculosis* DNA may be constructed in plasmid or phage lambda gt11 vector. The library of genomic fragments may be screened with genomic DNA of *Mycobacterium tuberculosis* as a probe to screen and select DNA fragments which rapidly hybridize to *Mycobacterium tuberculosis* genomic DNA. This may provide DNA fragments which hybridize very rapidly to *Mycobacterium tuberculosis* DNA. The DNA fragment may be incubated with clinical samples suspected of containing *Mycobacterium tuberculosis* of interest or may be incubated with *Mycobacterium tuberculosis* after it is cultured. If *Mycobacterium tuberculosis* is present, then the specific DNA fragment will hybridize with the DNA of *Mycobacterium tuberculosis* and can be detected by, for example, dot blot or Southern hybridization assay.

The nucleotide sequence of the specific DNA fragment may be determined and specific DNA primers may thus be designed. The so designed primers may be added to the clinical samples. If *Mycobacterium tuberculosis* is present in the clinical samples, the genomic DNA homologous to the specific DNA fragment may be amplified by PCR. The amplified product may be visualized by for example agarose gel electrophoresis and hybridization. The PCR amplification as well as hybridization with the specific DNA fragment of the invention may indicate that the sample contains *Mycobacterium tuberculosis* and hence may be used in detection and diagnosis of *tuberculosis*. In addition, the specific DNA fragment of the invention may be used to monitor the epidemiology of *tuberculosis* and strain polymorphism of *Mycobacterium tuberculosis* may be determined.

The method of this invention allows for rapid and specific diagnosis of tuberculosis and *Mycobacterium tuberculosis* infection. The assessment of the diagnosis with the DNA probe of this invention is accurate because the DNA probe owing to its sequence specificity only provides for hybridization and detection of *Mycobacterium tuberculosis* which indicates that the sample contains the mycobacteria of interest i.e. *Mycobacterium tuberculosis*.

The inventors have determined the nucleotide sequence of the DNA fragment of this invention which is 1291 bp long. The nucleotide sequence of the DNA fragment shows several interesting features including the presence of repeat sequences and an IS like sequence with an open reading frame. The IS like sequence is characterized by the presence of two inverted repeats flanked with direct repeat GTT on either side. GTT is a direct repeat which is located at 458 to 460 and at 1193 to 1195.

A section or fragment of the 1291 base pair sequence can also be used to detect *Mycobacterium tuberculosis.*

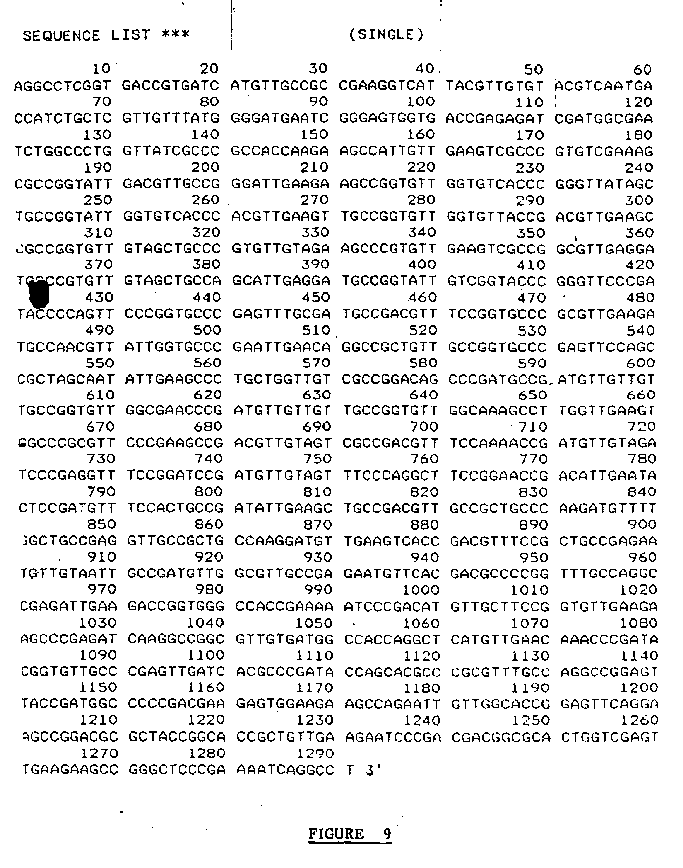

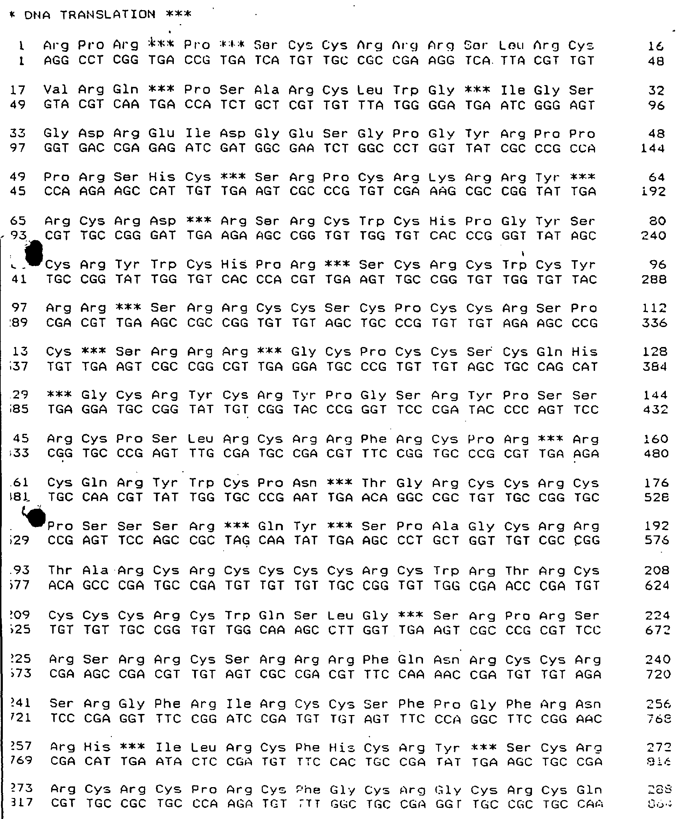

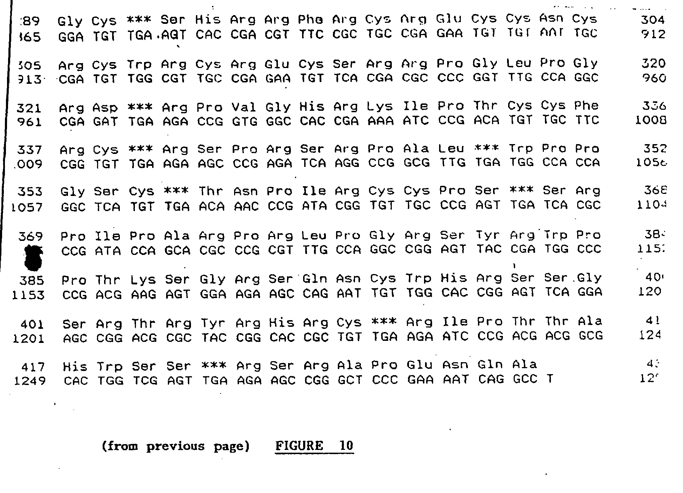

The deduced amino acid sequence from the nucleotide sequence described in Fig. 9 of the 1291 bp DNA fragment is shown in Fig. 10.

### I. Construction of a recombinant expression library of Mycobacterium tuberculosis DNA.

A recombinant DNA expression library of *Mycobacterium tuberculosis* DNA may be constructed using lambda gt11 vector. The library may be constructed with *Mycobacterium tuberculosis* genomic DNA fragments in such a way that all protein coding sequences would be represented and expressed (Young, R.A., B.R. Bloom, C.M. Grosskinsky, J. Ivyani, D. Thomas and R.W. Davis, Proceedings of the National Academy of Sciences, USA, 82:2583-2587 (1985)).

Lambda gtll is a bacteriophage vector which is capable of driving the expression of foreign insert DNA with E. coli transcription and translation signals. Lambda gtll expresses the insert DNA as a fusion protein connected to the E. coli betagalactosidase polypeptide. Lambda gtll and the E. coli strain used (Y1088 and Y1990) have been described previously (Young, R.A. and R. Davis. Proceedings of the National Academy of Sciences, USA, 80:1194-1198, 1983). The techniques of these publications are incorporated herein by reference. The library contained in this manner has a titer of 1 X 10¹⁰ pfu/ml. It contains approximately 60% recombinants with an average size of 4 kb.

### II. Screening of the lambda gt11 Mycobacterium tuberculosis library with genomic DNA of Mycobacterium tuberculosis as probe

Genomic DNA from *Mycobacterium tuberculosis* (*Mycobacterium tuberculosis* H37Rv and confirmed Indian clinical isolates) was used as a probe to screen the *Mycobacterium tuberculosis* recombinant DNA library. This work is described below and with specific reference to isolation of repetitive sequences.

The chromosomal DNA from *Mycobacterium tuberculosis* was labeled with digoxigenin and used as a probe. Screening of the lambda genomic library was performed as described in (Sambrook, H., E.F. Fritsch and T. Maniatis. 1989. Molecular Cloning, A Laboratory Manual, 2nd ed Cold Spring Harbor, NY).

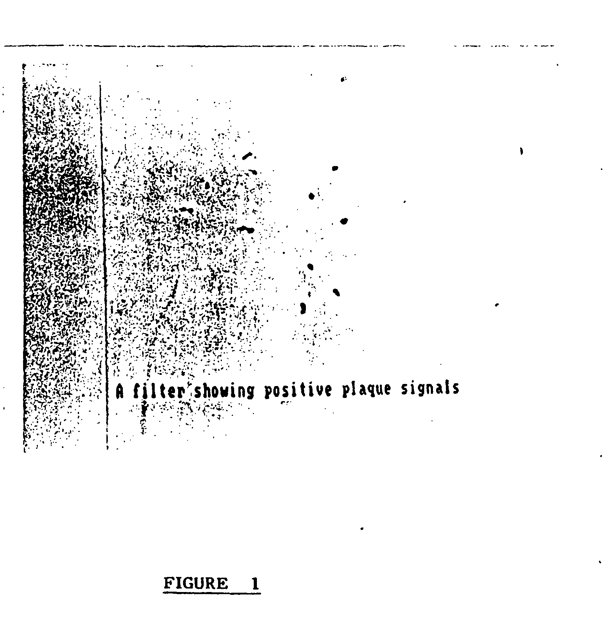

Briefly lambda gt11 recombinants were arrayed on a lawn of E. coli Y1088. The phages were grown and blotted onto nitrocellulose paper and probed with DIG labeled denatured chromosomal DNA from *Mycobacterium tuberculosis* by DNA hybridization. The signal producing plaques were detected with anti-DIG-alkaline phosphatase labeled antibodies according to the manufacturer's protocol (Boehringer Mannheim, Germany). DNA antibody conjugates were detected by alkaline phosphatase reaction with Nitro Blue tetrazolium (NBT) and 5-Bromo-4-Chloro-3-Indolyl Phosphate (BCIP). The positive hybridization was detected as blue plaque spots. Such signal producing clones were isolated in a screen of approximately 6 X 10⁴ recombinant plaques within 60 minutes of detection time (Fig.1).

### III. Probing of the Arrays of lambda gt11 DNA clones with chromosomal DNA as probe

0.3 ml of a saturated culture of Y1088 was mixed with recombinant phage (6,000 plaque forming units, pfu) and incubated at 36° C for 10 minutes. The mixture was added to 3 ml of molten LB soft agar and poured onto 90 mm plates containing 1.5% LB agar and allowed to harden at room temperature for 10 min. The plates were incubated at 37°C for a period at which point clear plaques approximately 1 mm in diameter were visible. The plates were then overlaid with nitrocellulose filters. Subsequent processing of filters for detection of clones containing repetitive sequences was identical to the procedures described for screening of a lambda gtll library with DNA probes (Davis, L.G., M.D. Dibner and J.F. Battey. Basic Methods in Molecular Biology, Elsevier. 1986). The nonradioactive DNA labeling and detection system of Boehringer Mannheim, Germany was used for labeling, hybridization and detection during screening. The amplified library was plated to an approximate 6 X 10³ pfu per plate using E. coli Y1088 as the host strain. Of total 60,000 recombinant plaques, 14 plaques were picked up as a result of tertiary screening with DIG-labeled denatured chromosomal DNA from *Mycobacterium tuberculosis* as a probe. These fourteen plaques gave positive signals within 60 minutes.

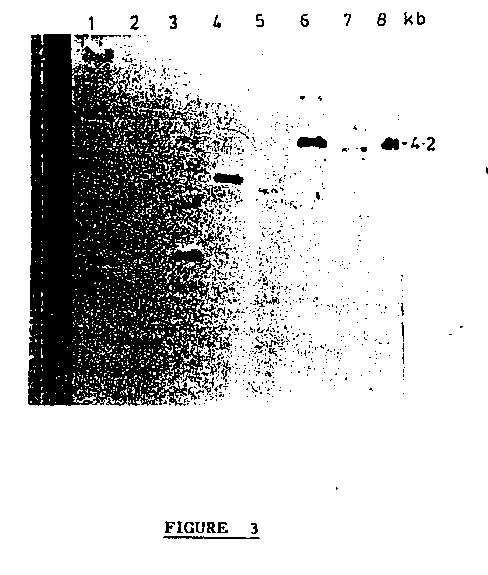

The recombinant plaques containing repetitive sequences specific to *Mycobacterium tuberculosis* were isolated by subtractive screening of 14 recombinant plaques with denatured chromosomal DNA from mycobacterial,strains other than *Mycobacterium tuberculosis* by DNA hybridization. This resulted in isolation of eight recombinant plaques which hybridized with *Mycobacterium tuberculosis* but not with denatured genomic DNA from *M*. *avium-intracellulare*, *M. fortuitum, M. chelonae, M. smegmatis* and *M. phlei*. These recombinant plaques were purified and the phage DNA was isolated and digested with EcoRI restriction enzyme to determine the size of the insert DNA in the recombinant plaques (Fig. 12 and Table I). The other set was subjected to Southern blotting and hybridization using DIG labeled denatured genomic DNA from *Mycobacterium tuberculosis*. Positive hybridization was detected within 60 minutes (Fig. 3).

All eight clones contained inserts of different sizes and hybridized with chromosomal DNA of *Mycobacterium tuberculosis*. The insert from clone C8 showed positive hybridization within 10 minutes of detection and was selected for detailed study.

**TABLE I**

| Recombinant clone | insert fragments (kb) |
|---|---|
| C8 | 4.2* kb |
| C10 | 3.8* kb |
| C16 | 4.2* kb |
| C33 | 2.7* kb |
| C38 | 3.0* kb |
| C41 | 2.4, 1.6*, 1.1, 0.7 kb |
| C60 | 3.8, 2.8, 0.76*, 0.64 kb |
| C70 | 2.4*, 1.6, 1.1, 0.7 kb |

| | |
|---|---|
| * The fragment(s) which gave positive hybridization signal with DIG-labelled denatured genomic DNA of *Mycobacterium tuberculosis* within 10-60 min. | |

### IV. Recombinant DNA manipulation

The clone C8 was selected. It contained an insert of 4.2 kb which gave strong hybridization signal within 10 minutes with DIG-labeled denatured chromosomal DNA from *Mycobacterium tuberculosis*. The 4.2 kb DNA fragment from clone C8 was isolated by restriction digestion and subsequent elution of the 4.2 kb fragment from the agarose gel. The 4.2 kb fragment was purified and subcloned into pUC13 plasmid vector. The resulting recombinant plasmid was designated as pC8.

### V. Localization of repetitive sequences within the 1291 bp StuI-StuI DNA Fragment

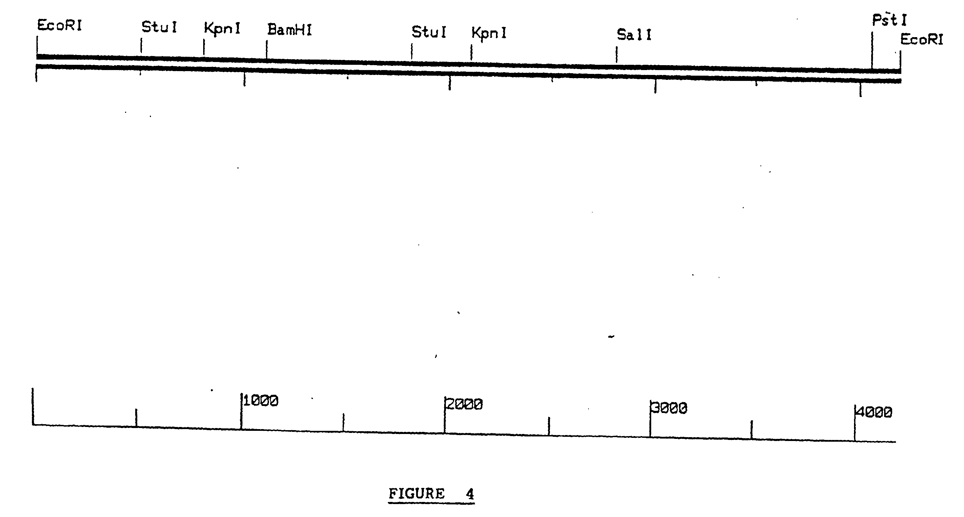

The restriction map of the 4.2 kb DNA fragment was determined (Fig. 4). It contained one site each for BamHI, SalI and two sites each for StuI and KpnI restriction enzymes.

The repetitive sequences within the 4.2 kb DNA fragment were further localized by hybridization of BamHI, SalI, StuI and KpnI restricted fragments of the 4.2 kb DNA fragment with DIG-labeled denatured genomic DNA of *Mycobacterium tuberculosis*.

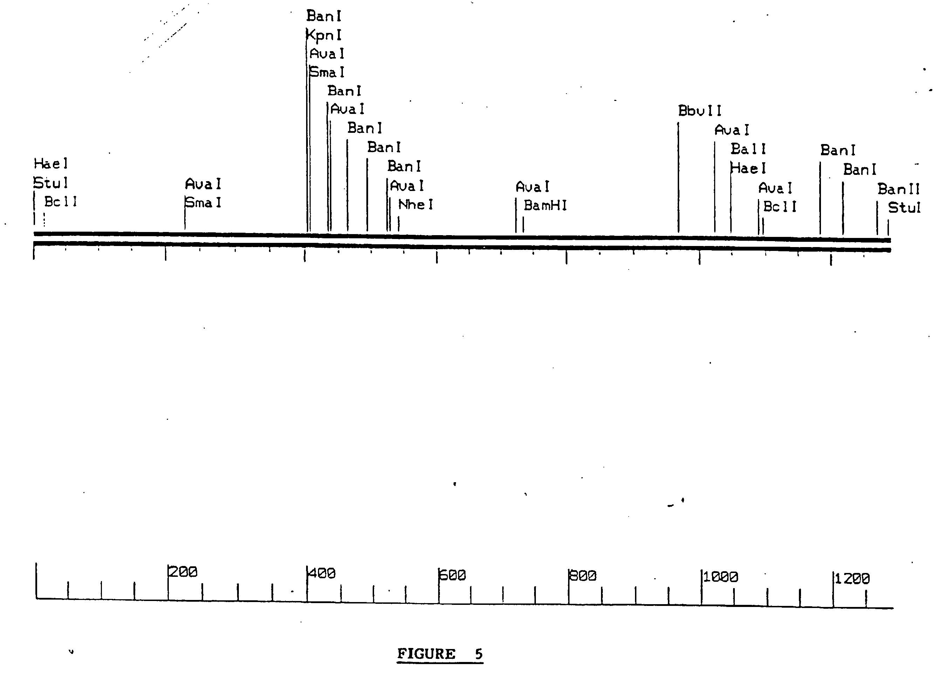

The repetitive sequences were localized within the StuI-StuI fragment present within the 4.2 kb DNA fragment cloned in this invention. The detailed restriction map of the StuI-StuI fragment was deduced from the nucleotide sequence of 1291 base pairs and confirmed by subsequent digestion with restriction enzymes (Fig 5).

### VI. Hybridization of the 1291 bp StuI-StuI DNA fragment with different mycobacterial strains and other pathogens

The genomic DNA from different mycobacteria and other pathogens was isolated and hybridized with the DIG-labeled 1291 bp DNA fragment of pC8 by Southern hybridization as well as dot blot hybridization. As shown in Fig. 6, the fragment did not hybridize in Southern hybridization to the following mycobacterial strains:
*M. smegmatis*
*M. phlei*
*M. fortuitum*
*M. chelonae*
*M. flavescens*
*M. chelonae*
*M. trivale*
*M. duvali*
*M. marinum*
*M. gordonae*
*M. kansasii*
*M. avium*
*M. intracellulare*
*M. scrofulaceum*
*M. gordonae*
*M. xenopi*
*M. aurum*
*M. microti*
*M. szulgai*

The genomic DNA from the above mentioned bacteria were isolated and digested with EcoRI restriction enzyme. The digest was electrophoresed in 0.8% agarose gel, blotted onto nitrocellulose paper and hybridized with the DIG-labeled denatured 1291 bp DNA fragment as a probe. The Southern hybridization of the 1291 bp fragment with genomic DNA of *Mycobacterium tuberculosis* revealed several bands confirming the presence of repetitive sequences within the 1291 bp DNA fragment whereas no hybridization was found with the other mycobacteria listed above (Figure 6).

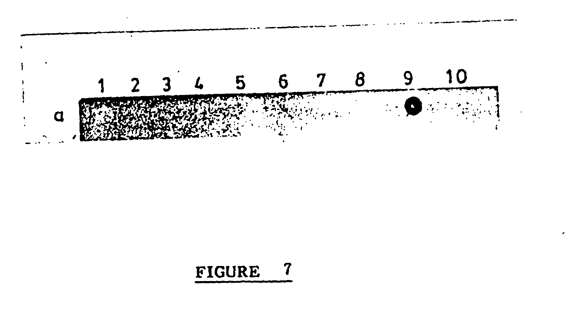

The hybridization of the 1291 bp DNA fragment was done with genomic DNA from other pathogenic organisms listed below. The result of dot blot hybridization is shown in Figure 7.
Salmonella typhimurium
Staphylococcus aureus
Proteus vulgaris
Pseudomonas aeruginosa
Klebsiella pneumoniae
Enterobacter alginii
vibrio cholerae
Bacillus subtilis
Edwardsiella
Salmon sperm DNA
Human placental DNA
E. coli

The 1291 bp DNA fragment did not hybridize to the genomic DNA of these pathogens (Figure 7).

### VII. Hybridization of the 1291 bp StuI-StuI DNA fragment with clinical isolates of Mycobacterium tuberculosis

The Southern hybridization of the 1291 bp DNA fragment with genomic DNA of clinical isolates of *Mycobacterium tuberculosis* should permit the detection of this 1291 bp DNA fragment in *Mycobacterium tuberculosis* and its use in epidemiology.

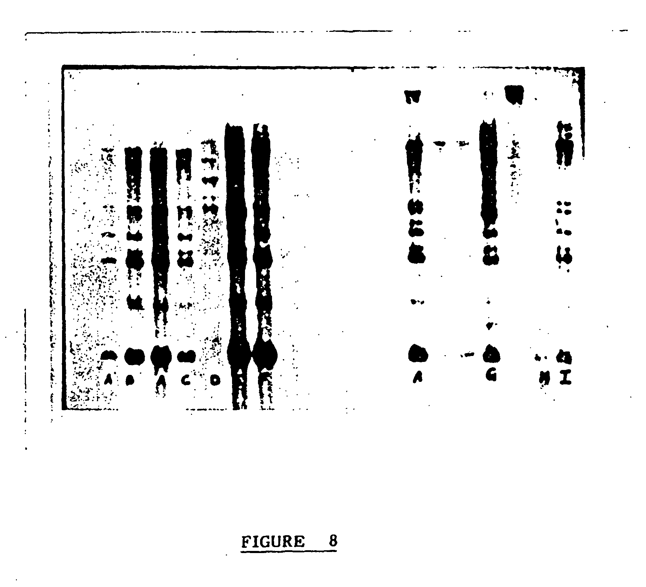

The genomic DNA from confirmed clinical isolates of *Mycobacterium tuberculosis* were isolated and digested with the StuI restriction enzyme. The digests were electrophoresed, transferred onto nitrocellulose paper and hybridized with the DIG-labeled denatured 1291 bp DNA fragment. The results with more than two hundred clinical isolates of *Mycobacterium tuberculosis* showed the presence of this fragment. The hybridization revealed several bands which confirmed the repetitive nature of the fragment. Several isotopes were identified, however all the isolates showed a strong band of 1291 bp (Figure 8).

### VIII. Nucleotide sequence of the 1291 bp StuI-StuI DNA fragment

The nucleotide sequence of the 1291 bp DNA fragment was determined by dideoxy chain termination sequencing technique (Biggin, M.D. et al. 1983. Proceedings of the National Academy of Sciences, USA. 80:3963-3965). The products of the sequencing reactions were electrophoresed on 6% acrylamide/ 7M urea/ 0.5-2.5 X TBE gradient sequencing gels. The gels were dried under vacuum and exposed to Kodak XRP-1 film. The nucleotide sequences were determined independently for both strands of the 1291 bp DNA fragment. Computer-aided analysis of the nucleotide sequence and deduced protein sequence was performed using databases and programs provided by the National Institute of Health, as well as the programs of Chou and Fasman and Hopp and Woods. (Chou, P.Y. and G.D. Fasman, Advances in Enzymol., 47:45-148, 1978; Hopp, T.P. and K.P. Woods, Proceedings of the National Academy of Sciences, USA, 78:3824-3828, 1981). The nucleotide sequence of the 1291 bp StuI-StuI region of the 4.2 kb DNA fragment is represented in Figure 9. The nucleotide sequence of the DNA fragment shows several interesting features including the presence of repeat sequences and an IS like sequence with open reading frame. The nucleotide sequence of the DNA fragment of this invention along with IS and repeat sequences present within the sequence can be seen in Figure 9. In Table II, the presence of direct repeats in the 1291 bp StuI-StuI DNA fragment is shown. Only 10 repeats of more than 9 nucleotide bases along with the sequence and their position have been shown. In Table III, the presence of the hairpin loop site in the IS like element present within the 1291 bp StuI-StuI DNA fragment has been shown. The two inverted repeats are flanked with direct repeat GTT on either side.

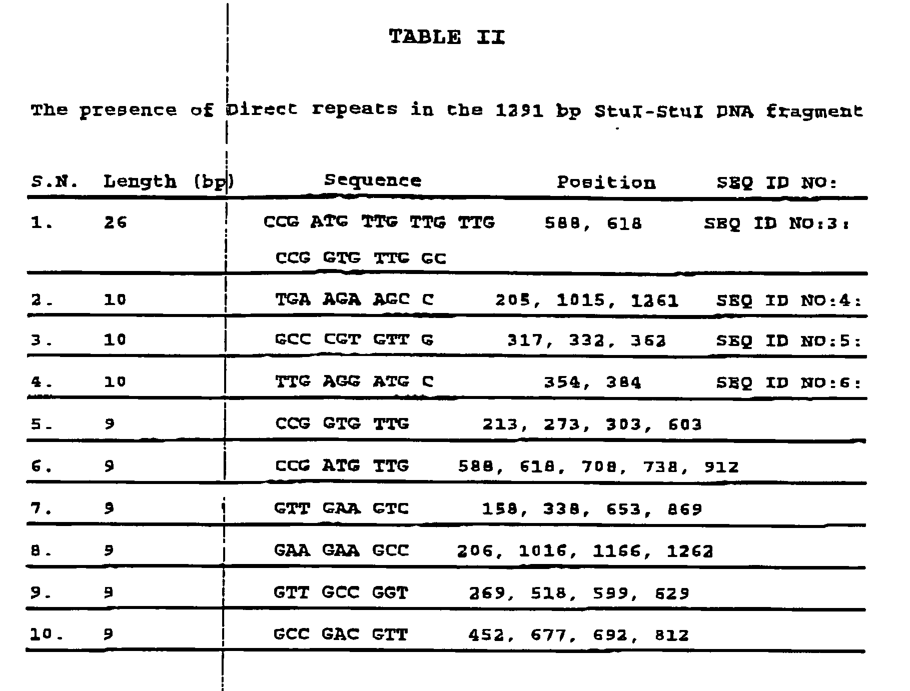

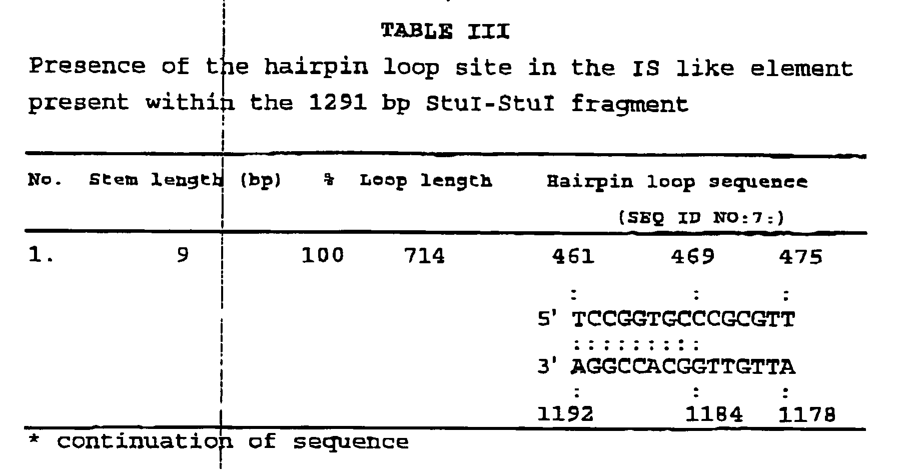

The inverted repeats are located at 461 to 469 (TCCGGTGCC) and at 1184 to 1192 (GGCACCGGA).

The deduced amino acid sequence from the nucleotide sequence described in Figure 9 of the 1291 bp DNA fragment is shown in Figure 10.

## Claims

1. Use of a nucleic acid molecule specific for *Mycobacterium tuberculosis* comprising the nucleotide sequence: 
AGGCCTCGGT GACCGTGATC ATGTTGCCGC CGAAGGTCAT TACGTTGTGT ACGTCAATGA
CCATCTGCTC GTTGTTTATG GGGATGAATC GGGAGTGGTG ACCGAGAGAT CGATGGCGAA
TCTGGCCCTG GTTATCGCCC GCCACCAAGA AGCCATTGTT GAAGTCGCCC GTGTCGAAAG
CGCCGGTATT GACGTTGCCG GGATTGAAGA AGCCGGTGTT GGTGTCACCC GGGTTATAGC
TGCCGGTATT GGTGTCACCC ACGTTGAAGT TGCCGGTGTT GGTGTTACCG ACGTTGAAGC
CGCCGGTGTT GTAGCTGCCC GTGTTGTAGA AGCCCGTGTT GAAGTCGCCG GCGTTGAGGA
TGCCCGTGTT GTAGCTGCCA GCATTGAGGA TGCCGGTATT GTCGGTACCC GGGTTCCCGA
TACCCCAGTT CCCGGTGCCC GAGTTTGCGA TGCCGACGTT TCCGGTGCCC GCGTTGAAGA
TGCCAACGTT ATTGGTGCCC GAATTGAACA GGCCGCTGTT GCCGGTGCCC GAGTTCCAGC
CGCTAGCAAT ATTGAAGCCC TGCTGGTTGT CGCCGGACAG CCCGATGCCG ATGTTGTTGT
TGCCGGTGTT GGCGAACCCG ATGTTGTTGT TGCCGGTGTT GGCAAAGCCT TGGTTGAAGT
[?]GCCCGCGTT CCCGAAGCCG ACGTTGTAGT CGCCGACGTT TCCAAAACCG ATGTTGTAGA
TCCCGAGGTT TCCGGATCCG ATGTTGTAGT TTCCCAGGCT TCCGGAACCG ACATTGAATA
CTCCGATGTT TCCACTGCCG ATATTGAAGC TGCCGACGTT GCCGCTGCCC AAGATGTTTT
GGCTGCCGAG GTTGCCGCTG CCAAGGATGT TGAAGTCACC GACGTTTCCG CTGCCGAGAA
TGTTGTAATT GCCGATGTTG GCGTTGCCGA GAATGTTCAC GACGCCCCGG TTTGCCAGGC
CGAGATTGAA GACCGGTGGG CCACCGAAAA ATCCCGACAT GTTGCTTCCG GTGTTGAAGA
AGCCCGAGAT CAAGGCCGGC GTTGTGATGG CCACCAGGCT CATGTTGAAC AAACCCGATA
CGGTGTTGCC CGAGTTGATC ACGCCCGATA CCAGCACGCC CGCGTTTGCC AGGCCGGAGT
TACCGATGGC CCCCGACGAA GAGTGGAAGA AGCCAGAATT GTTGGCACCG GAGTTCAGGA
AGCCGGACGC GCTACCGGCA CCGCTGTTGA AGAATCCCGA CGACGGCGCA CTGGTCGAGT
TGAAGAAGCC GGGCTCCCGA AAATCAGGCC T (SEQ ID NO. 1)
 or a sequence complementary thereto, or a sequence which hybridises with any of the aforesaid sequences by binding under non-stringent conditions (6X SSC, 50% formamide at room temperature) and washing under conditions of higher stringency (2X SSC, 65°C, where SSC = 0.15M NaCl, 0.015M sodium citrate, pH 7.2), or a fragment of SEQ ID NO. 1 specific for *Mycobacterium tuberculosis* for detecting and/or identifying *Mycobacterium tuberculosis* in a sample *ex vivo.*

2. Use as claimed in claim 1 wherein said nucleic acid molecule comprises the sequence: 
C GCGTTGAAGA 11
TGCCAACGTT ATTGGTGCCC GAATTGAACA GGCCGCTGTT GCCGGTGCCC GAGTTCCAGC 71
CGCTAGCAAT ATTGAAGCCC TGCTGGTTGT CGCCGGACAG CCCGATGCCG ATGTTGTTGT 131
TGCCGGTGTT GGCGAACCCG ATGTTGTTGT TGCCGGTGTT GGCAAAGCCT TGGTTGAAGT 191
CGCCCGCGTT CCCGAAGCCG ACGTTGTAGT CGCCGACGTT TCCAAAACCG ATGTTGTAGA 251
TCCCGAGGTT TCCGGATCCG ATGTTGTAGT TTCCCAGGCT TCCGGAACCG ACATTGAATA 311
CTCCGATGTT TCCACTGCCG ATATTGAAGC TGCCGACGTT GCCGCTGCCC AAGATGTTTT 371
GGCTGCCGAG GTTGCCGCTG CCAAGGATGT TGAAGTCACC GACGTTTCCG CTGCCGAGAA 431
TGTTGTAATT GCCGATGTTG GCGTTGCCGA GAATGTTCAC GACGCCCCGG TTTGCCAGGC 491
CGAGATTGAA GACCGGTGGG CCACCGAAAA ATCCCGACAT GTTGCTTCCG GTGTTGAAGA 551
AGCCCGAGAT CAAGGCCGGC GTTGTGATGG CCACCAGGCT CATGTTGAAC AAACCCGATA 611
CGGTGTTGCC CGAGTTGATC ACGCCCGATA CCAGCACGCC CGCGTTTGCC AGGCCGGAGT 671
TACCGATGGC CCCCGACGAA GAGTGGAAGA AGCCAGAATT GTT 714
(SEQ ID NO. 7)
 or a sequence complementary thereto, or a sequence which hybridises with any of the aforesaid sequences by binding under non-stringent conditions (6X SSC, 50% formamide at room temperature) and washing under conditions of higher stringency (2X SSC, 65°C, where SSC = 0.15M NaCl, 0.015M sodium citrate, pH 7.2).

3. Use as claimed in claim 1 wherein said nucleic acid molecule comprises one or more of the sequences of table II, or a sequence complementary thereto, or a sequence which hybridises with any of the aforesaid sequences by binding under non-stringent conditions (6X SSC, 50% formamide at room temperature) and washing under conditions of higher stringency (2X SSC, 65°C, where SSC = 0.15M NaCl, 0.015M sodium citrate, pH 7.2).

4. Use of as claimed in any one of claims 1 to 3 wherein said nucleic acid molecule is used as a probe.

5. Use as claimed in any one of claims 1 to 4 wherein said sample is selected from clinical isolates and body fluids.

6. Use as claimed in claim 5 wherein the clinical isolate sample is sputum, cerebrospinal fluid, pleural fluid, urine, gastric lavage, bronchial lavage, pericardial fluid or lymph node aspirate.

7. An *ex vivo* method of detecting *M*. *tuberculosis* in a sample wherein at least one nucleic acid molecule as defined in any one of claims 1 to 4 is used as a probe or a primer in a DNA-based detection system.

8. The method of claim 7 comprising incubating said probe nucleic acid molecule with samples which may contain specific mycobacteria *(M. tuberculosis)* and hybridization of nucleic acid with the DNA in the sample is detected.

9. An *ex vivo* method for the production and identification of a DNA fragment of *M. tuberculosis* comprising the 1291 base pair sequence as defined in claim 1 comprising the following steps:
(a) constructing a genomic library of *M*. *tuberculosis* in a vector;
(b) screening the genomic library of *M*. *tuberculosis* with genomic DNA of M. tuberculosis detectably labelled as a probe to select DNA fragments capable of specifically and rapidly hybridizing to *M*. *tuberculosis* genomic DNA;
(c) isolating clones including repetitive DNA fragments with positive hybridization signals appearing within 10 to 60 minutes.

10. An *ex vivo* method as claimed in claim 9 additionally comprising:
(d) further screening of the isolated clones for validation by DNA-DNA hybridization with genomic DNA of *mycobacteria* other than *M. tuberculosis* as probe to confirm that the isolated clone does not produce any hybridization signals with said other mycobacteria.

11. An *ex vivo* method as claimed in any one of claims 7 to 10 wherein the nucleic acid molecule or DNA fragment is hybridized to mycobacterial nucleic acid molecule or DNA by dot blot hybridization or southern hybridization or microplade hybridization.

12. A method as claimed in any one of claims 7 to 11, wherein the samples for *M. tuberculosis* are detected by hybridization and PCR amplification by adding primers selected for the nucleotide sequence of 1291 bp DNA fragment.

13. Use of the nucleic acid molecule as defined in any one of claims 1 to 4 for restriction fragment length polymorphism analysis of *M. tuberculosis* isolates.

14. A nucleic acid molecule specific for *M. tuberculosis* consisting of SEQ ID NO. 1, or a sequence complementary thereto, or a sequence having a sequence identity of 95% or more to any of the aforesaid sequences, or a fragment of SEQ ID NO. 1, wherein any such sequencer or fragment thereof is specific for *Mycobacterium tuberculosis*.

15. The nucleic acid molecule as claimed in claim 14 comprising the sequence of SEQ ID NO. 7 or one or more of the sequences of Table II, or a sequence complementary thereto.

## Patentansprüche

1. Verwendung eines für *Mycobacterium tuberculosis* spezifischen Nukleinsäuremoleküls, umfassend die Nukleotidsequenz: 
AGGCCTCGGT GACCGTGATC ATGTTGCCGC CGAAGGTCAT TACGTTGTGT ACGTCAATGA
CCATCTGCTC GTTGTTTATG GGGATGAATC GGGAGTGGTG ACCGAGAGAT CGATGGCGAA
TCTGGCCCTG GTTATCGCCC GCCACCAAGA AGCCATTGTT GAAGTCGCCC GTGTCGAAAG
CGCCGGTATT GACGTTGCCG GGATTGAAGA AGCCGGTGTT GGTGTCACCC GGGTTATAGC
TGCCGGTATT GGTGTCACCC ACGTTGAAGT TGCCGGTGTT GGTGTTACCG ACGTTGAAGC
CGCCGGTGTT GTAGCTGCCC GTGTTGTAGA AGCCCGTGTT GAAGTCGCCG GCGTTGAGGA
TGCCCGTGTT GTAGCTGCCA GCATTGAGGA TGCCGGTATT GTCGGTACCC GGGTTCCCGA
TACCCCAGTT CCCGGTGCCC GAGTTTGCGA TGCCGACGTT TCCGGTGCCC GCGTTGAAGA
TGCCAACGTT ATTGGTGCCC GAATTGAACA GGCCGCTGTT GCCGGTGCCC GAGTTCCAGC
CGCTAGCAAT ATTGAAGCCC TGCTGGTTGT CGCCGGACAG CCCGATGCCG ATGTTGTTGT
TGCCGGTGTT GGCGAACCCG ATGTTGTTGT TGCCGGTGTT GGCAAAGCCT TGGTTGAAGT
CGCCCGCGTT CCCGAAGCCG ACGTTGTAGT CGCCGACGTT TCCAAAACCG ATGTTGTAGA
TCCCGAGGTT TCCGGATCCG ATGTTGTAGT TTCCCAGGCT TCCGGAACCG ACATTGAATA
CTCCGATGTT TCCACTGCCG ATATTGAAGC TGCCGACGTT GCCGCTGCCC AAGATGTTTT
GGCTGCCGAG GTTGCCGCTG CCAAGGATGT TGAAGTCACC GACGTTTCCG CTGCCGAGAA
TGTTGTAATT GCCGATGTTG GCGTTGCCGA GAATGTTCAC GACGCCCCGG TTTGCCAGGC
CGAGATTGAA GACCGGTGGG CCACCGAAAA ATCCCGACAT GTTGCTTCCG GTGTTGAAGA
AGCCCGAGAT CAAGGCCGGC GTTGTGATGG CCACCAGGCT CATGTTGAAC AAACCCGATA
CGGTGTTGCC CGAGTTGATC ACGCCCGATA CCAGCACGCC CGCGTTTGCC AGGCCGGAGT
TACCGATGGC CCCCGACGAA GAGTGGAAGA AGCCAGAATT GTTGGCACCG GAGTTCAGGA
AGCCGGACGC GCTACCGGCA CCGCTGTTGA AGAATCCCGA CGACGGCGCA CTGGTCGAGT
TGAAGAAGCC GGGCTCCCGA AAATCAGGCC T (SEQ ID NO. 1)
 oder eine dazu komplementäre Sequenz, oder eine Sequenz, die mit einer der zuvor genannten Sequenzen durch Bindung unter nichtstringenten Bedingungen (6X SSC, 50 % Formamid, Raumtemperatur) und Waschung unter höherstringenten Bedingungen (2X SSC, 65°C, wobei SSC = 0,15 M NaCl, 0,015 M Natriumcitrat, pH 7,2) hybridisiert, oder ein für *Mycobacterium tuberculosis* spezifisches Fragment von SEQ ID NO:1, zur Detektion und/ oder Identifikation von *Mycobacterium tuberculosis* in einer *ex vivo-*Probe.

2. Verwendung nach Anspruch 1, wobei das Nukleinsäuremolekül die Sequenz: 
C GCGTTGAAGA 11
TGCCAACGTT ATTGGTGCCC GAATTGAACA GGCCGCTGTT GCCGGTGCCC GAGTTCCAGC 71
CGCTAGCAAT ATTGAAGCCC TGCTGGTTGT CGCCGGACAG CCCGATGCCG ATGTTGTTGT 131
TGCCGGTGTT GGCGAACCCG ATGTTGTTGT TGCCGGTGTT GGCAAAGCCT TGGTTGAAGT 191
CGCCCGCGTT CCCGAAGCCG ACGTTGTAGT CGCCGACGTT TCCAAAACCG ATGTTGTAGA 251
TCCCGAGGTT TCCGGATCCG ATGTTGTAGT TTCCCAGGCT TCCGGAACCG ACATTGAATA 311
CTCCGATGTT TCCACTGCCG ATATTGAAGC TGCCGACGTT GCCGCTGCCC AAGATGTTTT 371
GGCTGCCGAG GTTGCCGCTG CCAAGGATGT TGAAGTCACC GACGTTTCCG CTGCCGAGAA 431
TGTTGTAATT GCCGATGTTG GCGTTGCCGA GAATGTTCAC GACGCCCCGG TTTGCCAGGC 491
CGAGATTGAA GACCGGTGGG CCACCGAAAA ATCCCGACAT GTTGCTTCCG GTGTTGAAGA 551
AGCCCGAGAT CAAGGCCGGC GTTGTGATGG CCACCAGGCT CATGTTGAAC AAACCCGATA 611
CGGTGTTGCC CGAGTTGATC ACGCCCGATA CCAGCACGCC CGCGTTTGCC AGGCCGGAGT 671
TACCGATGGC CCCCGACGAA GAGTGGAAGA AGCCAGAATT GTT 714
(SEQ ID NO. 7)
 oder eine dazu komplementäre Sequenz oder eine Sequenz, die mit einer der zuvor genannten Sequenzen durch Bindung unter nichtstringenten Bedingungen (6X SSC, 50 % Formamid, Raumtemperatur) und Waschung unter höherstringenten Bedingungen (2X SSC, 65 °C, wobei SSC = 0,15 NaCl, 0,015 M Natriumcitrat, pH 7,2) hybridisiert, umfasst.

3. Verwendung nach Anspruch 1, wobei das Nukleinsäuremolekül eine oder mehrere der Sequenzen aus Tabelle II oder eine dazu komplementäre Sequenz oder eine Sequenz, die mit einer der zuvor genannten Sequenzen durch Bindung unter nichtstringenten Bedingungen (6X SSC, 50 % Formamid, Raumtemperatur) und Waschung unter höherstringenten Bedingungen (2X SSC, 65°C, wobei SSC = 0,15 M NaCl, 0,015 M Natriumcitrat, pH 7,2) hybridisiert, umfasst.

4. Verwendung nach einem der Ansprüche 1 bis 3, wobei das Nukleinsäuremolekül als Sonde verwendet wird.

5. Verwendung nach einem der Ansprüche 1 bis 4, wobei die Probe unter klinischen Isolaten und Körperflüssigkeiten ausgewählt ist.

6. Verwendung nach Anspruch 5, wobei die klinische Isolatprobe Sputum, Liquor cerebrospinalis, Pleuralflüssigkeit, Urin, Magenlavage, Bronchiallavage, Pericardflüssigkeit oder Lymphknotenaspirat ist.

7. Ex vivo-Verfahren zur Detektion von M. *tuberculosis* in einer Probe, wobei mindestens ein Nukleinsäuremolekül, wie in einem der Ansprüche 1 bis 4 definiert, in einem DNA-basierten Detektionssystem als Sonde oder Primer verwendet wird.

8. Verfahren nach Anspruch 7, umfassend eine Inkubation des Nukleinsäure-Sondenmoleküls mit Proben, die spezifische Mycobakterien *(M. tuberculosis)* enthalten können, und eine Detektion der Hybridisierung der Nukleinsäure mit der DNA in der Probe.

9. Ex vivo-Verfahren zur Herstellung und Identifikation eines die in Anspruch 1 definierte 1291-Basenpaar-Sequenz umfassenden DNA-Fragmentes von *M. tuberculosis*, umfassend die folgenden Schritte:
(a) Konstruktion einer genomischen Bibliothek von *M*. *tuberculosis* in einem Vektor;
(b) Durchmusterung der genomischen Bibliothek von *M*. *tuberculosis* mit als Sonde detektierbar markierter genomischer DNA von *M*. *tuberculosis* zur Selektion von DNA-Fragmenten, die spezifisch und schnell mit genomischer DNA von *M. tuberculosis* zu hybridisieren vermögen;
(c) Isolation von Klonen, die repetitive DNA-Fragmente mit positiven Hybridisierungssignalen, die innerhalb von 10 bis 60 Minuten erscheinen, enthalten.

10. Ex vivo-Verfahren nach Anspruch 9, weiterhin umfassend:
(d) weitere Durchmusterung der isolierten Klone zur Validierung durch DNA-DNA-Hybridisierung mit genomischer DNA von anderen Mycobakterien als *M*. *tuberculosis* als Sonde, um sicherzustellen, dass der isolierte Klon mit diesen anderen Mycobakterien keine Hybridisierungssignale erzeugt.

11. *Ex vivo*-Verfahren nach einem der Ansprüche 7 bis 10, wobei das Nukleinsäuremolekül oder DNA-Fragment durch Dot blot-Hybridisierung oder Southern-Hybridisierung oder Microplate-Hybridisierung mit einem mycobakteriellen Nukleinsäuremolekül hybridisiert wird.

12. Verfahren nach einem der Ansprüche 7 bis 11, wobei die Proben für *M. tuberculosis* durch Hybridisierung und PCR-Amplifikation durch Zusatz von für die Nukleotidsequenz des 1291 Basenpaar-DNA-Fragmentes ausgewählten Primern detektiert werden.

13. Verwendung des in einem der Ansprüche 1 bis 4 definierten Nukleinsäuremoleküls zur Restriktionsfragmentlängenmorphismus-Analyse von *M. tuberculosis-*Isolaten.

14. Für *M*. *tuberculosis* spezifisches Nukleinsäuremolekül, bestehend aus SEQ ID NO:1 oder einer dazu komplementären Sequenz oder einer Sequenz mit einer Sequenzidentität von 95 % oder mehr mit einer der zuvor genannten Sequenzen oder aus einem Fragment von SEQ ID NO:1, wobei jede solche Sequenz oder jedes solche Fragment für *Mycobacterium tuberculosis* spezifisch ist.

15. Nukleinsäuremolekül nach Anspruch 14, umfassend die Sequenz von SEQ ID NO:7 oder eine oder mehrere der Sequenzen der Tabelle II oder eine dazu komplementäre Sequenz.

## Revendications

1. Utilisation d'une molécule d'acide nucléique spécifique à Mycobacterium tuberculosis comprenant la séquence de nucléotide : 
AGGCCTCGGT GACCGTGATC ATGTTGCCGC CGAAGGTCAT TACGTTGTGT ACGTCAATGA
CCATCTGCTC GTTGTTTATG GGGATGAATC GGGAGTGGTG ACCGAGAGAT CGATGGCGAA
TCTGGCCCTG GTTATCGCCC GCCACCAAGA AGCCATTGTT GAAGTCGCCC GTGTCGAAAG
CGCCGGTATT GACGTTGCCG GGATTGAAGA AGCCGGTGTT GGTGTCACCC GGGTTATAGC
TGCCGGTATT GGTGTCACCC ACGTTGAAGT TGCCGGTGTT GGTGTTACCG ACGTTGAAGC
CGCCGGTGTT GTAGCTGCCC GTGTTGTAGA AGCCCGTGTT GAAGTCGCCG GCGTTGAGGA
TGCCCGTGTT GTAGCTGCCA GCATTGAGGA TGCCGGTATT GTCGGTACCC GGGTTCCCGA
TACCCCAGTT CCCGGTGCCC GAGTTTGCGA TGCCGACGTT TCCGGTGCCC GCGTTGAAGA
TGCCAACGTT ATTGGTGCCC GAATTGAACA GGCCGCTGTT GCCGGTGCCC GAGTTCCAGC
CGCTAGCAAT ATTGAAGCCC TGCTGGTTGT CGCCGGACAG CCCGATGCCG ATGTTGTTGT
TGCCGGTGTT GGCGAACCCG ATGTTGTTGT TGCCGGTGTT GGCAAAGCCT TGGTTGAAGT
[?]GCCCGCGTT CCCGAAGCCG ACGTTGTAGT CGCCGACGTT TCCAAAACCG ATGTTGTAGA
TCCCGAGGTT TCCGGATCCG ATGTTGTAGT TTCCCAGGCT TCCGGAACCG ACATTGAATA
CTCCGATGTT TCCACTGCCG ATATTGAAGC TGCCCACGTT GCCGCTGCCC AAGATGTTTT
GGCTGCCGAG GTTGCCGCTG CCAAGGATGT TGAAGTCACC GACGTTTCCG CTGCCGAGAA
TGTTGTAATT GCCGATGTTG GCGTTGCCGA GAATGTTCAC GACGCCCCGG TTTGCCAGGC
CGAGATTGAA GACCGCTGGG CCACCGAAAA ATCCCGACAT GTTGCTTCCG GTGTTGAAGA
AGCCCGAGAT CAAGGCCGGC GTTGTGATGG CCACCAGGCT CATGTTGAAC AAACCCGATA
CGGTGTTGCC CGAGTTGATC ACGCCCGATA CCAGCACGCC CGCGTTTGCC AGGCCGGAGT
TACCGATGGC CCCCGACGAA GAGTGGAAGA AGCCAGAATT GTTGGCACCG GAGTTCAGGA
AGCCGGACGC GCTAGCGGCA CCGCTGTTGA AGAATCCCGA CGACGGCGCA CTGGTCGAGT
TGAAGAAGCC GGGCTCCCGA AAATCAGGCC T (SEQ ID NO. 1)
 (séquence identifiée sous le n° 1)
ou une séquence qui en est complémentaire ou une séquence qui s'hybride avec l'une quelconque des séquences ci-dessus, par fixation dans des conditions non stringentes (6X SSC, formamide à 50 % à la température ambiante) et par lavage dans des conditions de stringence plus grandes (2X SSC, 65°C, avec SSC = 0,15M de NaCl, 0,015M de citrate de sodium, pH 7,2) ou un fragment de la séquence identifiée sous le n° 1 spécifique à Mycobacterium tuberculosis pour détecter et/ou identifier Mycobacterium tuberculosis dans un échantillon ex vivo.

2. Utilisation telle que revendiquée à la revendication 1, dans laquelle la molécule d'acide nucléique comprend la séquence : 
C GCGTTGAAGA 11
TGCCAACGTT ATTGGTGCCC GAATTGAACA GGCCGCTGTT GCCGGTGCCC GAGTTCCAGC 71
CGCTAGCAAT ATTGAAGCCC TGCTGGTTGT CGCCGGACAG CCCGATGCCG ATGTTGTTGT 131
TGCCGGTGTT GGCGAACCCG ATGTTGTTGT TGCCGGTGTT GGCAAAGCCT TGGTTGAAGT 191
CGCCCGCGTT CCCGAAGCCG ACGTTGTAGT CGCCGACGTT TCCAAAACCG ATGTTGTAGA 251
TCCCGAGGTT TCCGGATCCG ATGTTGTAGT TTCCCAGGCT TCCGGAACCG ACATTGAATA 311
CTCCGATGTT TCCAGTGCCG ATATTGAAGC TGCCGACGTT GCCGCTGCCC AAGATGTTTT 371
GGCTGCCGAG GTTGCCGCTG CCAAGGATGT TGAAGTCACC GACGTTTCCG CTGCCGAGAA 431
TGTTGTAATT GCCGATGTTG GCGTTGCCGA GAATGTTCAC GACGCCCCGG TTTGCCAGGC 491
CGAGATTGAA GACCGGTGGG CCACCGAAAA ATCCCGACAT GTTGCTTCCG GTGTTGAAGA 551
AGCCCGAGAT CAAGGCCGGC GTTGTGATGG CCACCAGGCT CATGTTGAAC AAACCCGATA 611
CGGTGTTGCC CGAGTTGATC ACGCCCGATA CCAGCACGCC CGCGTTTGCC AGGCCGGAGT 671
TACCGATGGC CCCCGACGAA GAGTGGAAGA AGCCAGAATT GTT 714
(SEQ ID NO. 7)
 (séquence identifiée sous le n° 7)
ou une séquence qui en est complémentaire ou une séquence qui s'hybride avec l'une quelconque des séquences ci-dessus, par fixation dans des conditions non stringentes (6X SSC, formamide à 50 % à la température ambiante) et par lavage dans des conditions de stringence plus grandes (2X SSC, 65°C, avec SSC = 0,15M de NaCl, 0,015M de citrate de sodium, pH 7,2).

3. Utilisation telle que revendiquée à la revendication 1, dans laquelle la molécule d'acide nucléique comprend une ou plusieurs des séquences du Tableau II ou une séquence qui en est complémentaire ou une séquence qui s'hybride avec l'une quelconque des séquences ci-dessus, par fixation dans des conditions non stringentes (6X SSC, formamide à 50 % à la température ambiante) et par lavage dans des conditions de stringence plus grandes (2X SSC, 65°C, avec SSC = 0,15M de NaCl, 0,015M de citrate de sodium, pH 7,2).

4. Utilisation telle que revendiquée dans l'une quelconque des revendications 1 à 3, dans laquelle la molécule d'acide nucléique est utilisée en tant que sonde.

5. Utilisation telle que revendiquée dans l'une quelconque des revendications 1 à 4, dans laquelle l'échantillon est choisi parmi des isolats cliniques et des fluides corporels.

6. Utilisation telle que revendiquée à la revendication 5, dans laquelle l'échantillon d'isolat clinique est du crachat, du fluide cérébrospinal, du fluide pleural, de l'urine, du lavage d'estomac, du lavage de bronches, du fluide péricardique, un échantillon prélevé par aspiration de ganglion lymphatique.

7. Procédé ex vivo de détection de M. tuberculosis dans un échantillon, dans lequel on utilise au moins une molécule d'acide nucléique telle que définie à l'une quelconque des revendications 1 à 4 comme sonde ou comme amorce dans un système de détection à base d'ADN.

8. Procédé suivant la revendication 7, dans lequel on fait incuber la molécule d'acide nucléique servant de sonde avec des échantillons qui peuvent contenir des mycobactéries spécifiques (M. tuberculosis) et l'on détecte une hybridation de l'acide nucléique avec l'ADN de l'échantillon.

9. Procédé ex vivo de production et d'identification d'un fragment d'ADN de M. tuberculosis comprenant la séquence de 1291 paires de base telle que définie à la revendication 1, comprenant les stades suivants :
(a) on construit une banque génomique de M. tuberculosis dans un vecteur ;
(b)on crible la banque génomique de M. tuberculosis par de l'ADN génomique de M. tuberculosis marqué de façon décelable sous la forme d'une sonde pour sélectionner des fragments d'ADN aptes à s'hybrider spécifiquement et rapidement à l'ADN génomique de M. tuberculosis ;
(c)on isole des clones comprenant des fragments répétitifs d'ADN ayant des signaux d'hybridation positifs apparaissant en 10 à 60 minutes.

10. Procédé ex vivo suivant la revendication 9, dans lequel, supplémentairement :
(d) on crible, en outre, les clones isolés pour la validation par hybridation ADN-ADN par de l'ADN génomique de mycobactéries autres que M. tuberculosis comme sonde afin de confirmer que le clone isolé ne produit pas de signal d'hybridation avec les autres mycobactéries.

11. Procédé ex vivo suivant l'une quelconque des revendications 7 à 10, dans lequel on hybride la molécule d'acide nucléique ou le fragment d'ADN à une molécule d'acide nucléique ou à de l'ADN mycobactérien par hybridation sur tache ou par hybridation Southern ou par hybridation sur microplaque.

12. Procédé ex vivo suivant l'une quelconque des revendications 7 à 11, dans lequel on détecte M. tuberculosis dans les échantillons par hybridation et amplification PCR en ajoutant des amorces choisies pour la séquence de nucléotide du fragment d'ADN à 1291 paire de base.

13. Utilisation de la molécule d'acide nucléique telle que définie à l'une quelconque des revendications 1 à 4 pour une analyse de polymorphisme de longueur de fragment par restriction d'isolats de M. tuberculosis.

14. Molécule d'acide nucléique spécifique à M. tuberculosis consistant en la séquence identifiée sous le n° 1 ou en une séquence qui en est complémentaire, ou en une séquence ayant une identité de séquence supérieure ou égale à 95 % de l'une des séquences précédentes ou en une fragment d'une séquence identifiée sous le n° 1, dans laquelle toute séquence de ce genre ou fragment de celle-ci est spécifique à Mycobacterium tuberculosis.

15. Molécule d'acide nucléique telle que revendiquée à la revendication 14, comprenant la séquence identifiée sous le n° 7 ou une ou plusieurs des séquences du Tableau II ou une séquence qui en est complémentaire.
